# EUROPEAN PATENT SPECIFICATION

(11) **EP 1 377 267 B1**
(45) Date of publication and mention of the grant of the patent: **08.03.2006**
(21) Application number: 01991346.6
(22) Date of filing: 19.12.2001
(51) Int. Cl.: A61K 8/86, A61K 8/891, A61Q 15/00

(54) **HIGH EFFICACY ANTIPERSPIRANT STICK WITH CONCENTRATED ELASTOMER**
HOCHAKTIVE ANTITRANSPIRANTSTIFTE ENTHALTEND EINEN KONZENTRIERTEN ELASTOMER
BATON ANTI-TRANSPIRATION TRES EFFICACE COMPRENANT UN ELASTOMERE CONCENTRE

(30) Priority: 21.12.2000 US 257269 P
(43) Date of publication of application: 07.01.2004
(73) Proprietor: Colgate-Palmolive Company, New York, N.Y. 10022 (US)
(72) Inventor: MATTAI, Jairajh, Piscataway, NJ 08854 (US); GUENIN, Eric, P., Pennington, NJ 08534 (US); LEE, Wilson, Bloomfield, NJ 07003 (US); HALL-PUZIO, Patricia, Succasunna, NJ 07876 (US); GALE, Anne, Landing, NJ 07850 (US)
(74) Representative: Prins, Adrianus Willem
(86) International application number: PCT/US2001/049229
(87) International publication number: WO 2002/049585

(56) References cited:
- EP-A- 0 953 335
- EP-A- 0 972 512
- WO-A-98/00097
- US-A- 5 648 066
- US-A- 5 654 362
- US-A- 5 942 215

## Description

### Field of the Invention

This invention relates to antiperspirant/deodorant stick products made without stearyl alcohol and which have higher efficacy and better aesthetics than stearyl alcohol gelled sticks.

### Background of the Invention

There is a continuing trend to develop new and superior cosmetic compositions especially for the reduction and/or elimination of wetness and/or odor under the arms. Particular efforts include developing lower residue products especially with improved efficacy and aesthetics. Various product forms have included sticks (especially gel/sticks), gels, soft solids, roll-ons, aerosols and creams. Of these various forms the sticlcs, gels, soft solids creams and roll-ons are made with a liquid base material incorporating a solidifying agent and/or gelling agent and/or thickening agent. Generally, these forms include a solution of the cosmetically active ingredient in a suitable vehicle, a suspension of the active ingredient in a carrier vehicle, or a multiphasic dispersion or emulsion in which a solution of the active ingredient is dispersed or suspended in some continuous phase or in which the solubilized active ingredient constitutes the continuous phase.

One of the most frequently used gelling agents for stick products is stearyl alcohol. While it gives a solid product, it can reduce efficacy of the antiperspirant salt included in the formulation. This invention is a stick made without stearyl alcohol and which has an efficacy that is at least 10% better in sweat reduction than a stick that is gelled with stearyl alcohol.

Thus, it is an object of the invention to provide improved cosmetic compositions with the improvements as previously described and which are useful as antiperspirants and/or deodorants. These and other objects of the invention will be apparent from the following description of the invention.

### Summary of the Invention

It has been found that a high efficacy antiperspirant/deodorant stick product may be made by combining the following ingredients where all amounts are in weight percent based on the total weight of the composition:
(a) 30-70% volatile cyclomethicone (particularly 40-50%) (for example, D4, D5, D6 and mixtures of two or more of the foregoing);
(b) 10-25% of an antiperspirant active;
(c) 1-15% of an emollient (which may also be a mixture of two or more emollients) and which may include a non-volatile silicone (especially wherein the emollient is selected from the group consisting of C12-15 allyl benzoate; and medium volatility dimethicone (especially 10-350 centistoke material and more especially 10-50 centistoke material);
(d) 1-14% of polyethylene (particularly 3-10%) comprising one or more members selected from the group consisting of homopolymers and copolymers of polyethylene wherein the polyethylene (i) is at least 90% linear; (ii) has a molecular weight in the range of 300-3000 (especially 300-1000 and more especially 300-500); (iii) has a melting point in the range of 50-129 degrees C (for example, 50-70 degrees C, 60-70 degrees C, and 70-129 degrees C); and (iv) has a polymer backbone of CH₃CH₂-(CH₂-CH₂)ₙ-CH₂-CH3 (which can also be represented as CH₃CH₂-(CH₂-CH₂)ₙ-H), where n is an average number and is selected to be in the range of 10-106 (for example, polyethylenes sold under the PERFORMALENE name from New Phase Technology, Piscataway, NJ);
(e) 0.3-7% of a wax (including a single wax or a combination of waxes) as a co-gellant with the polyethylene wherein the wax has a melting point in the range of 40-97 degrees C (for example, 40-80 degrees C), and particularly wherein the wax is a member selected from the group consisting of Japan wax substitute 525 (from Ross Wax, Jersey City, NJ), Beeswax 136 (for example, from Ross Wax); microcrystalline wax having a melting point in the range of 60-97 degrees C.; and
(f) 1-30% (particularly 5-20%) of an elastomer in cyclomethicone composition comprising a cyclomethicone (and) dimethicone crosspolymer made with an ≡Si-H containing polysiloxane and an alpha, omega-diene of formula CH₂=CH(CH₂)ₓCH=CH₂, where x=1-20, to form a gel by crosslinking and addition of ≡Si-H across double bonds in the alpha, omega diene, which crosspolymer has a viscosity in the range of 50,000-3,000,000 centipoise (particularly 100,000-1,000,000; more particularly 250,000-450,000 centipoise; and most particularly 350,000 centipoise), preferably with a nonvolatiles content of 8-18% (particularly 10-14% and most particularly 12-13%) in cyclomethicone (for example a D4 or D5 cyclomethicone), (an example of such a crosspolymer composition being DC-9040 from Dow Corning Corporation (Midland, MI) with other types of such crosspolymers (also called elastomers) being described in U.S: Patent 5,654,362; provided that the ratio of wax:polyethylene is in the range of 1:1-1:10, particularly 1:2-1:10, and more particularly in a ratio of 3:8.

Other optional ingredients include 0.1-5% fragrance and an effective amount of an antimicrobial (for example, an antibacterial) agent.

### Detailed Description of the Invention

Various types of cyclomethicones may be used. Illustratively, and not by way of limitation, the volatile silicones are one or more members selected from the group consisting of cyclic polydimethylsiloxanes such as those represented by Formula I: 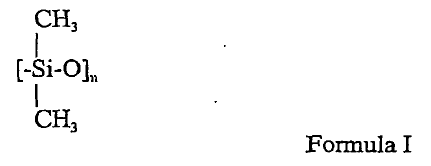
where n is an integer with a value of 3-7, particularly 5-6. By volatile silicone material is meant a material that has a measurable vapor pressure at ambient temperature. For example, DC-245 fluid or DC-345 fluid from Dow Coming Corporation (Midland, Michigan) is a type of cyclomethicone which can be used. These include a tetramer (or octylmethylcyclotetrasiloxane) and a pentamer (or decamethylcyclopentasiloxane).

The antiperspirant active can be selected from the group consisting of any of the known antiperspirant active materials. These include, by way of example (and not of a limiting nature), aluminum chlorohydrate, aluminum chloride, aluminum sesquichlorohydrate, zirconyl hydroxychloride, aluminum-zirconium glycine complex (for example, aluminum zirconium trichlorohydrex gly, aluminum zirconium pentachlorohydrex gly, aluminum zirconium tetrachlorohydrex gly and aluminum zirconium octochlorohydrex gly), aluminum chlorohydrex PG, aluminum chloxohydrex PEG, aluminum dichlorohydrex PG, and aluminum dichlorohydrex PEG. The aluminum-containing materials can be commonly referred to as antiperspirant active aluminum salts. Generally, the foregoing metal antiperspirant active materials are antiperspirant active metal salts. In the embodiments which are antiperspirant compositions according to the present invention, such compositions need not include aluminum-containing metal salts, and can include other antiperspirant active materials, including other antiperspirant active metal salts. Generally, Category I active antiperspirant ingredients listed in the Food and Drug Administration's Monograph on antiperspirant drugs for over-the-counter human use can be used. In addition, any new drug, not listed in the Monograph, such as aluminum nitratohydrate and its combination with zirconyl hydroxychlorides and nitrides, or aluminum-stannous chlorohydrates, can be incorporated as an antiperspirant active ingredient in antiperspirant compositions according to the present invention.

Particular types of antiperspirant actives include aluminum zirconium trichlorohydrex and aluminum zirconium tetrachlorohydrex either with or without glycine. A particular antiperspirant active is aluminum trichlorohydrex gly such as AZZ-902 SUP (from Reheis Inc., Berkley Heights, NJ) which has 98% of the particles less than 10 microns in size.

Antiperspirant actives can be incorporated into compositions according to the present invention in amounts in the range of 10-25% (on an actives basis) of the final composition, but the amount used will depend on the formulation of the composition. At lower levels the antiperspirant active material will not substantially reduce the flow of perspiration as effectively, but will reduce malodor, for example, by acting also as an antimicrobial material.

The antiperspirant active material is desirably included as particulate matter suspended in the composition of the present invention in amounts as described above, but can also be added as solutions or added directly to the mixture.

Emollients are a known class of materials in this art, imparting a soothing effect to the skin. These are ingredients which help to maintain the soft, smooth, and pliable appearance of the skin. Emollients are also known to reduce whitening on the skin and/or improve aesthetics. Examples of chemical classes from which suitable emollients can be found include:
(a) fats and oils which are the glyceryl esters of fatty acids, or triglycerides, normally found in animal and plant tissues, including those which have been hydrogenated to reduce or eliminate unsaturation. Also included are synthetically prepared esters of glycerin and fatty acids. Isolated and purified fatty acids can be esterified with glycerin to yield mono-, di-, and triglycerides. These are relatively pure fats which differ only slightly from the fats and oils found in nature. The general structure may be represented by Formula III: 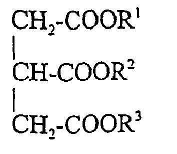 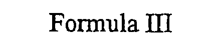
   wherein each of R¹, R², and R³ may be the same or different and have a carbon chain length (saturated or unsaturated) of 7 to 30. Specific examples include peanut oil, sesame oil, avocado oil, coconut, cocoa butter, almond oil, safflower oil, corn oil, cotton seed oil, castor oil, hydrogenated castor oil, olive oil, jojoba oil, cod liver oil, palm oil, soybean oil, wheat germ oil, linseed oil, and sunflower seed oil;
(b) hydrocarbons which are a group of compounds containing only carbon and hydrogen. These are derived from petrochemicals. Their structures can vary widely and include aliphatic, alicyclic and aromatic compounds. Specific examples include paraffin, petrolatum, hydrogenated polyisobutene, and mineral oil.
(c) esters which chemically, are the covalent compounds formed between acids and alcohols. Esters can be formed from almost all acids (carboxylic and inorganic) and any alcohol. Esters here are derived from carboxylic acids and an alcohol. The general structure would be R⁴CO-OR⁵. The chain length for R⁴ and R⁵ can vary from 7 to 30 and can be saturated or unsaturated, straight chained or branched. Specific examples include isopropyl myristate, isopropyl palmitate, isopropyl stearate, isopropyl isostearate, butyl stearate, octyl stearate, hexyl laurate, cetyl stearate, diisopropyl adipate, isodecyl oleate, diisopropyl sebacate, isostearyl lactate, C₁₂₋₁₅ alkyl benzoates, myreth-3 myristate, dioctyl malate, neopentyl glycol diheptanoate, neopentyl glycol dioctanoate, dipropylene glycol dibenzoate, C₁₂₋₁₅ alcohols lactate, isohexyl decanoate, isohexyl caprate, diethylene glycol dioctanoate, octyl isononanoate, isodecyl octanoate, diethylene glycol diisononanoate, isononyl isononanoate, isostearyl isostearate, behenyl behenate, C ₁₂₋₁₅ alkyl fumarate, laureth-2 benzoate, propylene glycol isoceteth-3 acetate, propylene glycol ceteth-3 acetate, octyldodecyl myristate, cetyl ricinoleate, myristyl myristate.
(d) saturated and unsaturated fatty acids which are the carboxylic acids obtained by hydrolysis of animal or vegetable fats and oils. These have general structure R⁶COOH with the R⁶ group having a carbon chain length between 7-10 straight chain.
(e) saturated and unsaturated fatty alcohols (including guerbet alcohols) with general structure R⁷COH where R⁷ can be straight chain and have carbon length of 7 to 10.
(f) lanolin and its derivatives which are a complex esterified mixture of high molecular weight esters of (hydroxylated) fatty acids with aliphatic and alicyclic alcohols and sterols. General structures would include R⁸CH₂-(OCH₂CH₂)ₙOH where R⁸ represents the fatty groups derived from lanolin and n=5 to 75 or R⁹CO-(OCH₂CH₂)ₙOH where R⁹CO- represents the fatty acids derived from lanolin and n=5 to 100. Specific examples include lanolin, lanolin oil, lanolin wax, lanolin alcohols, lanolin fatty acids, isopropyl lanolate, ethoxylated lanolin and acetylated lanolin alcohols.
(g) alkoxylated alcohols wherein the alcohol portion is selected from aliphatic alcohols having 2-18 and more particularly 4-18 carbons, and the alkylene oxide portion is selected from the group consisting of ethylene oxide, and propylene oxide having a number of alkylene oxide units from 2-53 and, more particularly, from 2-15. Specific examples include PPG-14 butyl ether and PPG-53 butyl ether.
(h) silicones as the linear organo-substituted polysiloxanes which are polymers of silicon/oxygen with general structure:
   (1) (R¹⁰)₃SiO(Si (R¹¹)₂O)ₓSi(R¹²)₃ where R¹⁰, R¹¹ and R¹² can be the same or different and are each independently selected from the group consisting of phenyl and C1-C60 alkyl; or
   (2) HO(R¹⁴)₂SiO(Si(R¹⁵)₂O)ₓSi(R¹⁶)₂OH, where R¹⁴, R¹⁵ and R¹⁶ can be the same or different and are each independently selected from the group consisting of phenyl and C1-C60 alkyl; (with specific examples including dimethicone, dimethiconol behenate, C₃₀₋₄₅ alkyl methicone, stearoxytrimethylsilane, phenyl trimethicone and stearyl dimethicone);
(i) mixtures and blends of two or more of the foregoing.

One particular group of emollients includes C12-15 alkyl benzoate (FINSOLV TN from Finetex Inc., Elmwood Park, NJ), medium volatility dimethicone (especially 10-350 centistoke material and more especially 10-50 centistoke material), isopropyl myristate; and neopentyl glycol diheptanoate.

The emollient or emollient mixture or blend thereof incorporated in compositions according to the present invention can, illustratively, be included in amounts of 1-15%, and particularly 3 - 12 % by weight of the total weight of the composition.

Polyethylenes may be made in a variety of ways. Each polymerization method has its own advantages and disadvantages and may e used to obtain a polymer with specific properties. For example, free radical polymerization of ethylene using radical initiators usually gives highly branched polymers known as low-density polyethylene. This method usually requires high temperatures and pressures. Preparation of linear polyethylene can be achieved at low temperatures and pressures using transition metal compounds and organometallic compounds as a catalyst. Zeigler-Natta catalyst (for example, TiCl₄ and Al(C₂H₅)₃) is a widely used catalyst system for commercial preparation of linear polyethylene. The molecular weight of the polymer can be manipulated by controlling temperature, pressure and the ratios of the two-part catalyst system used. The molecular weight can also be controlled by using chain transfer agents such as molecular hydrogen and Zn(C₂H₅)₂. Active hydrogen compounds (for example, methanol) can also bring about termination of the growing chains just as they do in anionic polymerization.

The method for making both low and high molecular weight linear polyethylene is the same. Low molecular weight polymer is obtained by controlling the molecular weight using chain transfer agents such a hydrogen gas or methanol followed by isolation of the desired molecular weight through fractionation by distillation or reprecipitation with solvents of varying polarities. One can also use a catalyst system which employs a combination of transition metal compound or an element from Groups IV to VIII such as vanadium, chromium, or cobalt as well as an organometallic compound of a metal from Groups I and III of the periodic table. One typical example for making linear polyethylene is described below (see Example PE).

The polyethylenes useful in this invention include those sold under the PERFORMALENE™ product line (New Phase Technology, Piscataway, NJ); MARCUS polyethylenes (for example M200, M300, M500 and M4040) (Marcus Oil and Chemical, Houston, Texas); HPWax polyethylene waxes (for example, HP CWP 200, HP CWP 500 and HP 400M) (Hase Petroleum Wax Co., Arlington Heights, IL). Mixtures of neutral polyethylene wax/polypropylene wax may also be used such as Polarwachs® PT30, Polarwachs® PT70, and Polycerit® AT-grades (TH. C. TROMM GmbH, Germany). Suitable polyethylenes may also be made using information found in the art such as British Patent 1 450 285.

One particular elastomer of interest is DC-9040 from the Dow Coming Corporation (Midland, MI).

The stick antiperspirant/deodorant products of this invention is an opaque product which leaves little or no white residue when applied and which exhibits improved efficacy and stability as compared to other stick formulations made with stearyl alcohol. Reduction of sweat of at least 10% more than that achieved with sticks gelled with stearyl alcohol can be achieved with the compositions of the invention.

Suitable antibacterial or antimicrobial agents include, for example, bacteriostatic quaternary ammonium compounds such as 2-amino-2-methyl-1-propanol (AMP), cetyltrimethylammonium bromide, cetyl pyridinium chloride, 2, 4, 4'-trichloro-2'-hydroxydiphenylether (Triclosan), N-(4-chlorophenyl)-N'-(3,4-dichlorophenyl)urea (Triclocarban), silver halides, octoxyglycerin (Sensiva™ SC 50) and various zinc salts (for example, zinc ricinoleate). The bacteriostat can, illustratively, be included in the composition in an amount of 0-5%, particularly 0.01-1.0% by weight, of the total weight of the composition. Triclosan, can illustratively be included in an amount of from 0.05% to about 0.5% by weight, of the total weight of the composition.

A variety of fragrances can be used in these compositions if scented products are desired. Fragrances can be used in an amount in the range of 0-5%, particularly 0.01--2.0%, and, for example, at a level of 1%.

Masking agents can be used in an amount of 0.05-5.0% (particularly 0.45-2%) by weight based on the total weight of the composition if an unscented product is desired.

For additional hardening of sticks, other additives having a melting point in the range of 78-98 degrees C such as long chain alcohols (such as Performacol 350 (having an average carbon chain length of 24 carbons), Performacol 425 (having an average carbon chain length of 30 carbons), or Performacol 550 (having an average carbon chain length of 40 carbons)); alcohol ethoxylates (such as Performathox 420 (20% by weight ethoxylation) and Performathox 450 (50% by weight ethoxylation) all available from New Phase Technology, Piscataway, NJ may be used.

For reducing whitening in sticks liquid or solid high refractive index materials may be used such as diethylhexyl 2,6-naphthalate (from C.P. Hall Co., Chicago, IL) or phenyltrimethicone (from Dow Coming Corp., Midland, MI) as well as other suitable ingredients.

Other various optional components include those described in U.S. Patents Numbers 5,019,375 to Tanner et al; 4,937,069 to Shin; and 5,102,656 . Examples of such additional ingredients include fragrances, coloring agents, opacificers, etc. in types and amounts conventionally used for such products.

These compositions are sticks made as suspensions and thickened or gelled by the combination of polyethylene and selected wax components.

The products of the invention can be made by conventional mixing techniques. The emollients are selected, weighed out and heated with stirring to about 65 degrees C. Next the wax component is added and heating is continued to a temperature in the range of 82-85 degrees C. The polyethylene is added. The mixture is cooled to about 80 degrees C and the elastomer plus additional cyclomethicone (which has been preheated to about 70 degrees C) is added. The mixture is cooled further to 75 degrees C. and the antiperspirant active is added. The temperature is increased to about 80 degrees C and held there for about 10 minutes with mixing. Fragrance, an antibacterial agent, coloring, etc. are then added if desired and thoroughly mixed. The final mixture is poured into suitable containers and then passed through a cooling tunnel which is at about 4 degrees C. or placed in a refrigerator for a suitable length of time on a laboratory scale. Cooling is then completed (completion of cooling can also be done at room temperature).

The composition can be rubbed onto the skin from the top surface of the container (itself fed from a reservoir of product in the container) so as to deposit an adequate amount of the cosmetic composition on to the skin. The cosmetic composition, for example, an antiperspirant/deodorant may be applied to the skin in the axillary regions to deposit sufficient amounts of antiperspirant and/or deodorant active material to reduce body malodor and/or reduce perspiration in axillary regions of the human body.

Various forms of the invention can be exemplified by the following formulations but should not be construed as limitations on the invention:

### EXAMPLES

The following Examples are offered as illustrative of the invention and are not to be construed as limitations thereon. In the Examples and elsewhere in the description of the invention, chemical symbols and terminology have their usual and customary meanings. In the Examples as elsewhere in this application (a) values for n, m, etc. in formulas, molecular weights and degree of ethoxylation or propoxylation are averages; (b) temperatures are in degrees C unless otherwise indicated; and (c) the amounts of the components are in weight percents based on the standard described; if no other standard is described then the total weight of the composition is to be inferred. Various names of chemical components include those listed in the CTFA International Cosmetic Ingredient Dictionary (Cosmetics, Toiletry and Fragrance Association, Inc., 7^{th} ed. 1997). Mixing techniques used to make the compositions are those conventionally used in the art including those described above.

### Example PE

A 3-liter flask reactor is equipped with a manometer and stirring apparatus and is set at atmospheric pressure with constant stirring. The reactor temperature is set at 65 degrees C by thermostat, purged with nitrogen, purged with ethylene, and then charged with 1 liter of purified dry cyclohexane, 4.6 millimoles of TiCl₄ , and 2.0 millirnoles of Al(C₂H₅)₃. Ethylene is then fed at the rate of 1 liter/minute into the reactor. After 15 minutes, the reaction is quenched by bubbling hydrogen gas through the reaction mixture. The low molecular weight polymers (which are oligomers) are separated by fractional distillation of the product mixture at reduced pressure (200 Torr, 26,664 Pascals).

### Example 1: General Method of Making Compositions

The emollients (for example, dimethicone (for example, DC-200, 10 centistokes and/or DC-200 350 centistokes from Dow Corning Corp.)) and C12-15 alkyl benzoate (FINSOLV TN brand product) are weighed out and placed in a 600 ml beaker. Each of the other ingredients is weighed out separately. Heating with stirring is initiated for the emollients in the 600 ml beaker until the temperature is about 65 degrees. C. The wax component is then added (for example, Japan Wax Sub 525 and/ or microcrystalline wax from Ross). Heating and stirring are continued until the temperature is in the range of 82-85 degrees C. The polyethylene (for example, PERFORMALENE-400 from New Phase Technology, Piscataway, NJ) is then added with stirring. The mixture is cooled to about 80 degrees and the elastomer (DC-9040 from Dow Corning) plus additional cyclomethicone (DC-345 from Dow Coming Corp.) which has been preheated to about 70 degrees C is then added with stirring. The mixture is further cooled to about 75 degrees C and the antiperspirant active salt (for example, Reach AZZ 902 SUF aluminum zirconium salt or Reach AZP 908 from Reheis Inc., Berkeley Heights, NJ) is added with mixing. The temperature is increased to about 80 degrees C and held there for about 10 minutes with mixing. Fragrance is added and mixing is continued for 1 minute. The mixture is poured into oval containers of the type normally used for antiperspirant/deodorant products and placed in a refrigerator at about 4 degrees C for about 15 minutes. Cooling is completed at room temperature.

In some of the examples additional ingredients such as diethylhexyl 2,6-naphthalate or Performacol 350 alcohol can be added.

## Claims

1. A high efficacy stick antiperspirant/deodorant free of added stearyl alcohol comprising in weight percent based on the total weight of the composition:
(a) 30-70% volatile cyclomethicone;
(b) 10-25% of an antiperspirant active;
(c) 1-15% of an emollient;
(d) 1-14% of polyethylene comprising one or more members selected from the group consisting of homopolymers and copolymers of polyethylene wherein the polyethylene (i) is at least 90% linear; (ii) has a molecular weight in the range of 300-3000; (iii) has a melting point in the range of 50-129 degrees C; and (iv) has a polymer backbone of CH₃CH₂-(CH₂-CH₂)ₙ-CH₂-CH₃, where n is an average number and is selected to be in the range of 10-106;
(e) 0.3-7% of a wax as a co-gellant with the polyethylene wherein the wax has a melting point in the range of 40-97 degrees C; and
(f) 1-30% of an elastomer in cyclomethicone composition comprising a cyclomethicone (and) dimethicone crosspolymer made with an ≡Si-H containing polysiloxane and an alpha, omega-diene of formula CH₂=CH(CH₂)ₓCH=CH₂ , where x=1-20, to form a gel by crosslinking and addition of ≡Si-H across double bonds in the alpha, omega diene, which crosspolymer has a viscosity in the range of 50,000-3,000,000 centipoise;
provided that the ratio of wax:polyethylene is in the range of 1:1-1:10.

2. A stick as claimed in Claim 1 comprising 40-50% of a volatile silicone.

3. A stick as claimed in Claim 1 wherein the emollient comprises a mixture of two or more emollients.

4. A stick as claimed in Claim 1 wherein the emollient is a member of the group consisting of
(a) fats and oils represented by Formula III: 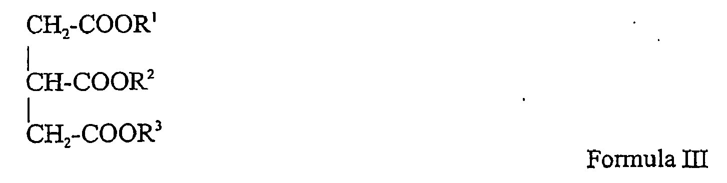
wherein each of R¹, R², and R³ may be the same or different and have a carbon chain length (saturated or unsaturated) of 7 to 30;
(b) hydrocarbons selected from the group consisting of paraffin, petrolatum, hydrogenated polyisobutene, and mineral oil;
(c) esters of general structure would be R⁴CO-OR⁵ wherein the chain length for R⁴ and R⁵ hydrocarbon groups is in the range of 7-30 and can be saturated or unsaturated, straight chained or branched;
(d) saturated and unsaturated fatty acids which have general structure R⁶COOH with the R⁶ group being a straight chain hydrocarbon with a carbon chain length between 7-10;
(e) saturated and unsaturated fatty alcohols which have a general structure R⁷COH where R⁷ is a straight chain hydrocarbon with a carbon length of 7 to 10;
(f) lanolin and its derivatives selected from the group consisting of lanolin, lanolin oil, lanolin wax, lanolin alcohols, lanolin fatty acids, isopropyl lanolate, ethoxylated lanolin and acetylated lanolin alcohols;
(g) alkoxylated alcohols wherein the alcohol portion is selected from aliphatic alcohols having 2-18 carbons, and the alkylene oxide portion is selected from the group consisting of ethylene oxide, and propylene oxide having a number of alkylene oxide units from 2-53;
(h) silicones as the linear organo-substituted polysiloxanes which are polymers of silicon/oxygen with general structure:
(1) (R¹⁰)₃SiO(Si(R¹¹)₂O)ₓSi(R¹²)₃ where R¹⁰, R¹¹ and R¹² can be the same or different and are each independently selected from the group consisting of phenyl and C1-C60 alkyl; or
(2) HO(R¹⁴)₂SiO(Si(R¹⁵)₂O)ₓSi(R¹⁶)₂OH, where R¹⁴, R¹⁵ and R¹⁶ can be the same or different and are each independently selected from the group consisting of phenyl and C1-C60 alkyl; and
(i) mixtures and blends of two or more of the foregoing.

5. A stick as claimed in Claim 1 comprising 3-12 % emollient.

6. A stick as claimed in Claim 1 wherein the emollient comprises a non-volatile silicone.

7. A stick as claimed in Claim 6 wherein the emollient comprises a 10-350 centistoke dimethicone.

8. A stick as claimed in Claim 1 comprising 3-10% polyethylene.

9. A stick as claimed in Claim 1 wherein the polyethylene has a melting point in the range of 50-70 degrees C.

10. A stick as claimed in Claim 1 wherein the polyethylene has a melting point in the range of 60-70 degrees C.

11. A stick as claimed in Claim 1 wherein the polyethylene has a melting point in the range of 70-129 C.

12. A stick as claimed in Claim 1 wherein the wax has a melting point in the range of 40-80 degrees C.

13. A stick as claimed in Claim 1 wherein the wax is a microcrystalline wax having a melting point in the range of 60-97 degrees C.

14. A stick as claimed in Claim 1 comprising 5-20% elastomer.

15. A stick as claimed in Claim 1 additionally comprising an effective amount of an antimicrobial agent.

## Patentansprüche

1. Hochwirksamer Antitranspirant/Deodorant-Stift, der frei von zugesetztem Stearylalkohol ist und in Gew.-%, bezogen auf das Gesamtgewicht der Zusammensetzung, umfasst:
(a) 30 bis 70 % flüchtiges Cyclomethicon,
(b) 10 bis 25 % Antitranspirantwirkstoff,
(c) 1 bis 15 % Aufweichmittel,
(d) 1 bis 14 % Polyethylen, das ein oder mehrere Mitglieder ausgewählt aus der Gruppe bestehend aus Homopolymeren und Copolymeren von Polyethylen umfasst, wobei das Polyethylen (i) zu mindestens 90 % linear ist, (ii) ein Molekulargewicht im Bereich von 300 bis 3000 aufweist, (iii) einen Schmelzpunkt im Bereich von 50 bis 129 °C aufweist, und (iv) ein Polymergerüst aus CH₃-CH₂₋(CH₂-CH₂)ₙ-CH₂-CH₃ besitzt, wobei n eine durchschnittliche Zahl ist und so gewählt ist, dass sie im Bereich von 10 bis 106 liegt,
(e) 0,3 bis 7 % Wachs als Co-Geliermittel mit dem Polyethylen, wobei das Wachs einen Schmelzpunkt im Bereich von 40 bis 97 °C aufweist, und
(f) 1 bis 30 % Elastomer in Cyclomethiconzusammensetzung, die ein Cyclomethicon (und) Dimethicon-Kreuzpolymer umfasst, das mit einem ≡Si-H enthaltenden Polysiloxan und einem α,ω-Dien der Formel CH₂=CH(CH₂)ₓCH=CH₂ hergestellt worden ist, in der x 1 bis 20 ist, um durch Vernetzung und Addition von ≡Si-H über Doppelbindungen in dem α,ω-Dien ein Gel zu bilden, wobei das Kreuzpolymer eine Viskosität im Bereich von 50 000 bis 3 000 000 Centipoise aufweist, mit der Maßgabe, dass das Verhältnis Wachs:Polyethylen im Bereich von 1 : 1 bis 1 : 10 liegt.

2. Stift nach Anspruch 1, der 40 bis 50 % flüchtiges Silikon umfasst.

3. Stift nach Anspruch 1, bei dem das Aufweichmittel eine Mischung aus zwei oder mehr Aufweichmitteln umfasst.

4. Stift nach Anspruch 1, bei dem das Aufweichmittel ein Mitglied aus der Gruppe bestehend aus
(a) Fetten und Ölen mit der Formel III: 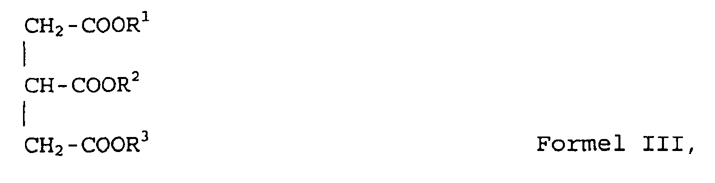 in der jedes von R¹, R² und R³ gleich oder verschieden sein kann und eine Kohlenstoffkettenlänge (gesättigt oder ungesättigt) von 7 bis 30 aufweist,
(b) Kohlenwasserstoffen ausgewählt aus der Gruppe bestehend aus Paraffin, Petrolatum, hydriertem Polyisobuten und Mineralöl,
(c) Estern der allgemeinen Struktur R⁴CO-OR⁵, in der die Kettenlänge für die R⁴- und R⁵-Kohlenwasserstoffgruppen im Bereich von 7 bis 30 liegt und diese gesättigt oder ungesättigt, geradkettig oder verzweigt sein kann,
(d) gesättigten und ungesättigten Fettsäuren mit der allgemeinen Struktur R⁶COOH, in der die R⁶-Gruppe ein geradkettiger Kohlenwasserstoff mit einer Kohlenstoffkettenlänge von 7 bis 10 ist,
(e) gesättigten und ungesättigten Fettalkoholen, die eine allgemeine Struktur R⁷COH aufweisen, in der R⁷ ein geradkettiger Kohlenwasserstoff mit einer Kohlenstofflänge von 7 bis 10 ist,
(f) Lanolin und seinen Derivaten ausgewählt aus der Gruppe bestehend aus Lanolin, Lanolinöl, Lanolinwachs, Lanolinalkoholen, Lanolinfettsäuren, Isopropyllanolat, eth-oxyliertem Lanolin und acetylierten Lanolinalkoholen,
(g) alkoxylierten Alkoholen, in denen der Alkoholanteil aus aliphatischen Alkoholen mit 2 bis 18 Kohlenstoffatomen ausgewählt ist und der Alkylenoxidanteil aus der Gruppe bestehend aus Ethylenoxid und Propylenoxid mit einer Zahl von Alkylenoxideinheiten von 2 bis 53 ausgewählt ist,
(h) Silikonen wie den linearen organosubstituierten Polysiloxanen, die Polymere von Silikon/Sauerstoff mit der allgemeinen Struktur:
(1) (R¹⁰)₃SiO(Si(R¹¹)₂O)ₓSi(R¹²)₃, in der R¹⁰, R¹¹ und R¹² gleich oder verschieden sein können und jeweils unabhängig ausgewählt sind aus der Gruppe bestehend aus Phenyl und C₁- bis C₆₀-Alkyl, oder
(2) HO(R¹⁴)₂SiO(Si(R¹⁵)₂O)ₓSi(R¹⁶)₂OH, in der R¹⁴, R¹⁵ und R¹⁶ gleich oder verschieden sein können und jeweils ausgewählt sind aus der Gruppe bestehend aus Phenyl und C₁- bis C₆₀-Alkyl, und
(i) Mischung oder Gemischen von zwei oder mehr der zuvor Genannten ist.

5. Stift nach Anspruch 1, der 3 bis 12 % Aufweichmittel umfasst.

6. Stift nach Anspruch 1, bei dem das Aufweichmittel ein nicht-flüchtiges Silikon umfasst.

7. Stift nach Anspruch 6, bei dem das Aufweichmittel ein Dimethicon mit 10 bis 350 Centistokes umfasst.

8. Stift nach Anspruch 1, der 3 bis 10 % Polyethylen umfasst.

9. Stift nach Anspruch 1, bei der das Polyethylen einen Schmelzpunkt im Bereich von 50 bis 70 °C aufweist.

10. Stift nach Anspruch 1, bei dem das Polyethylen einen Schmelzpunkt im Bereich von 60 bis 70 °C aufweist.

11. Stift nach Anspruch 1, bei dem das Polyethylen einen Schmelzpunkt im Bereich von 70 bis 129 °C aufweist.

12. Stift nach Anspruch 1, bei dem das Wachs einen Schmelzpunkt im Bereich von 40 bis 80 °C aufweist.

13. Stift nach Anspruch 1, bei dem das Wachs ein mikrokristallines Wachs mit einem Schmelzpunkt im Bereich von 60 bis 97 °C ist.

14. Stift nach Anspruch 1, der 5 bis 20 % Elastomer umfasst.

15. Stift nach Anspruch 1, der zusätzliche eine wirksame Menge antimikrobielles Mittel umfasst.

## Revendications

1. Un bâton de déodorant/anti-transpirant d'une grande efficacité ne contenant pas d'alcool stéarique et comprenant en pourcentage en poids par rapport au poids total de la composition :
(a) de 30 à 70% de cyclométhicone volatile ;
(b) de 10 à 25% d'un actif anti-transpirant ;
(c) de 1 à 15% d'un émollient ;
(d) de 1 à 14% de polyéthylène comprenant un ou plusieurs éléments choisis dans le groupe se composant d'homopolymères et de copolymères de polyéthylène dans lequel le polyéthylène (i) est linéaire au moins à 90% ; (ii) a un poids moléculaire se situant dans un intervalle de 300 à 3000 ; (iii) a un point de fusion se situant dans un intervalle de 50 à 129 degrés C ; et (iv) a une chaîne principale polymère de CH₃CH₂-(CH₂-CH₂)ₙ-CH₂-CH₃, dans laquelle n est un nombre moyen et est choisi pour qu'il se situe dans un intervalle de 10 à 106 ;
(e) de 0,3 à 7% d'une cire en tant que co-gélifiant avec le polyéthylène dans laquelle la cire a un point de fusion se situant dans un intervalle de 40 à 97 degrés C ; et
(f) de 1 à 30% d'un élastomère dans la composition de cyclométhicone comprenant un polymère réticulé cyclométhicone (et) diméthicone fait avec un polysiloxane contenant un =Si-H et un alpha, oméga-diène ayant pour formule CH₂=CH(CH₂)ₓCH=CH₂ dans laquelle x = de 1 à 20, pour constituer un gel par la réticulation et l'addition de ≡Si-H à travers des liaisons doubles dans l'alpha, oméga-diène, ledit polymère réticulé ayant une viscosité se situant dans un intervalle de 50 000 à 3 000 000 de centipoises ;
à condition que la proportion de cire/polyéthylène se situe dans un intervalle de 1/1 à 1/10.

2. Un bâton selon la revendication 1 comprenant de 40 à 50% d'un silicone volatile.

3. Un bâton selon la revendication 1 dans lequel l'émollient comprend un mélange de deux ou plusieurs émollients.

4. Un bâton selon la revendication 1 dans lequel l'émollient est un élément du groupe se composant :
(a) de graisses et d'huiles représentées par la Formule III : 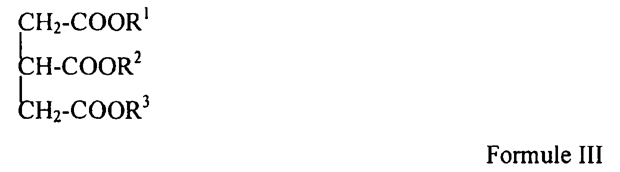
dans laquelle chacun de R¹, R² et R³ peut être le même ou différent et a une longueur de chaîne carbonée (saturée ou insaturée) de 7 à 30 ;
(b) d'hydrocarbures choisi dans le groupe se composant de la paraffine, de la vaseline, du polyisobutène hydrogéné et de l'huile minérale ;
(c) d'esters dont la structure générale serait R⁴CO-OR⁵ dans laquelle la longueur de la chaîne des groupes hydrocarbures R⁴ et R⁵ se situe dans un intervalle de 7 à 30 et peut être saturée ou insaturée, à chaîne linéaire ou ramifiée ;
(d) d'acides gras saturés et insaturés ayant une structure générale R⁶COOH, le groupe R⁶ étant un hydrocarbure à chaîne linéaire ayant une longueur de chaîne carbonée de 7 à 10 ;
(e) d'alcools gras saturés et insaturés ayant une structure générale R⁷COH dans laquelle R⁷ est un hydrocarbure à chaîne linéaire ayant une longueur de chaîne carbonée de 7 à 10 ;
(f) de lanoline et de ses dérivés choisis dans le groupe se composant de la lanoline, de l'huile de lanoline, de la cire de lanoline, des alcools de lanoline, des acides gras de lanoline, du lanolate d'isopropyle, de la lanoline éthoxylée et des alcools de la lanoline acétylée ;
(g) d'alcools alkoxylés dans lesquels la partie alcool est choisie parmi les alcools aliphatiques ayant de 2 à 18 carbones et la partie oxyde d'alkylène est choisie dans le groupe se composant de l'oxyde d'éthylène et de l'oxyde de propylène ayant un nombre d'unités d'oxydes d'alkylène de 2 à 53 ;
(h) de silicones comme les polysiloxanes organo-substitués qui sont des polymères de silicone/oxygène ayant la structure générale suivante :
(1) (R¹⁰)₃-SiO(Si(R¹¹)₂O)₂Si(R¹²)₃ dans laquelle R¹⁰, R¹¹ et R¹² peuvent être les mêmes ou différents et sont chacun indépendamment choisis dans le groupe se composant de phényle et d'alkyle en C₁ à C₆₀ ; ou
(2) HO(R¹⁴)₂SiO(Si(R¹⁵)₂O)₄Si(R¹⁶)₂OH, dans laquelle R¹⁴, R¹⁵ et R¹⁶ peuvent être les mêmes ou différents et sont chacun indépendamment choisis dans le groupe se composant de phényle et d'alkyle en C₁ à C₆₀ ; et
(i) de mélanges de deux ou de plusieurs des produits ci-dessus mentionnés.

5. Un bâton selon la revendication 1 comprenant de 3 à 12 % d'émollient.

6. Un bâton selon la revendication 1 dans lequel l'émollient comprend un silicone non volatile.

7. Un bâton selon la revendication 6 dans lequel l'émollient comprend un diméthicone de 10 à 350 centistokes.

8. Un bâton selon la revendication 1 comprenant de 3 à 10% de polyéthylène.

9. Un bâton selon la revendication 1 dans lequel le polyéthylène a un point de fusion se situant dans un intervalle de 50 à 70 degrés C.

10. Un bâton selon la revendication 1 dans lequel le polyéthylène a un point de fusion se situant dans un intervalle de 60 à 70 degrés C.

11. Un bâton selon la revendication 1 dans lequel le polyéthylène a un point de fusion se situant dans un intervalle de 70 à 129 degrés C.

12. Un bâton selon la revendication 1 dans lequel la cire a un point de fusion se situant dans un intervalle de 40 à 80 degrés C.

13. Un bâton selon la revendication 1 dans lequel la cire est une cire microcristalline ayant un point de fusion se situant dans un intervalle de 60 à 97 degrés C.

14. Un bâton selon la revendication 1 comprenant de 5 à 20% d'élastomère.

15. Un bâton selon la revendication 1 comprenant en outre une quantité efficace d'un agent antimicrobien.
